# EUROPEAN PATENT SPECIFICATION

(11) **EP 3 426 017 B1**
(45) Date of publication and mention of the grant of the patent: **11.08.2021**
(21) Application number: 16728818.2
(22) Date of filing: 17.06.2016
(51) Int. Cl.: A01G 31/00, A61K 36/59, A61K 8/9789, A61Q 19/08

(54) **HYDROPONIC TILIACORA TRIANDRA AND USE THEREOF**
HYDROPONISCHE TILIACORA TRIANDRA UND VERWENDUNG DAVON
TILIACORA TRIANDRA HYDROPONIQUE ET SON UTILISATION

(30) Priority: 04.03.2016 US 201662303480 P
(43) Date of publication of application: 16.01.2019
(73) Proprietor: Avon Products, Inc., Suffern, NY 10901 (US)
(72) Inventor: HWANG, Cheng S., New Milford, New Jersey 07646 (US); LYGA, John W., Basking Ridge, New Jersey 07920 (US)
(74) Representative: Abel & Imray
(86) International application number: PCT/US2016/037995
(87) International publication number: WO 2017/151174

(56) References cited:
- US-A- 4 075 785
- US-A- 4 118 891
- US-A1- 2006 150 497
- US-A1- 2010 166 677
- US-A1- 2012 003 332
- US-A1- 2013 283 689
- PAPAKONSTANTINOU ET AL.: 'Hyaluronic acid A key molecule in skin aging' DERMATO-ENDOCRINOLOGY vol. 4, no. 3, July 2012, XP055415127
- HAMMOND: 'The Avon Lady comes collecting Asian medicinal plants' TWN THIRD WORLD NETWORK, [Online] May 2012, pages 1 - 6, XP055093354 Retrieved from the Internet: <URL:www.twnside.org.sg>

## Description

### FIELD OF INVENTION

The invention relates generally to extracts of hydroponically grown plants of the *Tiliacora* genus, and the use of such extracts in skincare compositions and associated methods for reducing dermatological signs of aging and/or improving the health of human skin.

### BACKGROUND

Numerous skincare products have been developed for improving the appearance of human skin. Many topically applied skin care compositions contain plant extracts as active agents for imparting various functional attributes. One such plant that has found use in skincare is *Tiliacora triandra* Diels of the *Tiliacora* family, also known as Yanang, which is a species of flowering plant native to mainland Southeast Asia and used particularly in the cuisines of northeast Thailand and Laos. It is a climbing plant with mostly single, smooth, oval-shaped, deep green leaves and yellowish flowers. U.S. Patent Nos. 8,751,758 and 9,084,744, both titled, "Use of *Tiliacora triandra* in cosmetics and compositions thereof," disclose extracts of *Tiliacora triandra* that enhance fibroblasts and keratinocytes survival, and protect against collagen degradation. U.S. Patent No. 9,238,000 discloses an extract of *Tiliacora triandra* that upregulates WIPI-1 in fibroblasts and, consequently, is said to be useful for treatment of signs of skin aging caused by reduced autophagy activity. The foregoing patents do not disclose hydroponically grown *Tiliacora* plants.

It is an object of the present invention to provide hydroponically grown plants of the *Tiliacora* genus, including *Tiliacora triandra,* as well as skincare compositions comprising such extracts, and associated methods of topical application to human skin to improve health and combat dermatological manifestations of skin aging and damage. It is another object of the present invention to provide extracts from plants of the *Tiliacora* genus, including *Tiliacora triandra,* that are effective to elevate hyaluronic acid (HA) levels in the skin. It is yet another object of the present invention to provide skin care compositions comprising effective amounts of extracts of hydroponically grown plants of the *Tiliacora* genus, including *Tiliacora triandra,* in combination with effective amounts of glycolic acid and/or niacinamide, and methods of topical application to human skin to improve health and combat dermatological manifestations of skin aging and damage.

The foregoing discussion is presented solely to provide a better understanding of nature of the problems confronting the art and should not be construed in any way as an admission as to prior art nor should the citation of any reference herein be construed as an admission that such reference constitutes "prior art" to the instant application.

### SUMMARY OF INVENTION

In accordance with one or more of the foregoing objectives and others, the present invention provides hydroponically grown plants of the *Tiliacora* genus, including *Tiliacora triandra,* and extracts of such plants for skincare. Without being bound by any particular theory, it is believed that the extracts according to the invention are distinguished by their ability to stimulate or enhance production of the glycosaminoglycan (GAG) hyaluronic acid (HA) in fibroblast cells. HA is synthesized by dermal fibroblasts and epidermal keratinocytes. HA has a unique capacity to bind and retain water molecules and contributes to the hydration and resilient properties of skin. The skin content of HA decreases with age, which is one factor believed to account for the striking alterations in aged skin, such as loss of skin moisture and suppleness, as well as the development of wrinkles and fine lines. Accordingly, in some implementation of the invention, the extracts of plants of the *Tiliacora* genus, including *Tiliacora triandra,* are characterized by the ability to stimulate HA production in human fibroblasts and/or keratinocytes.

In one aspect of the invention, an extract of a hydroponically grown *Tiliacora triandra* plant *(e.g.,* whole plant) or portion thereof *(e.g.,* vines, leaves, flowers, etc.) is provided. In some implementations, the extract is prepared from aerial portions of the plant, such as vines, leaves, and/or flowers. The extract may be obtained using any known methodology, including steam distillation or water extraction, but is typically obtained by extraction with an organic solvent, such as a polar *(e.g.,* ethanol, etc.) or non-polar solvent (*e.g.*, hexanes) and/or a protic (*e.g.,* alcohols, acids, etc.) or non-protic solvent *(e.g.,* dialkyl ethers, alkanes, esters, etc.). In some implementations, the extraction is carried out using a polar protic solvent (*e.g.*, ethanol) alone or in combinations with water or other organic solvents. In a particular implementation, the extract is prepared by extraction of the plant or a portion thereof with a water/ethanol extraction solvent, for example, a solvent system comprising from 5-50% (v/v) water and from 50-95% (v/v) ethanol, or comprising from 10-30% (v/v) water and from 70-90% (v/v) ethanol (*e.g.*, a 20/80 (v/v) water/ethanol solvent system).

In some implementations, an extract of a *Tiliacora triandra* plant or portion thereof, characterized by the ability of a 0.2% (w/w) solution of said extract to enhance levels of hyaluronic acid in fibroblast cells by more than 30%. For example, it is contemplated that extracts which enhance levels of hyaluronic acid in fibroblast cells by more than 40%, more than 50%, more than 60%, more than 70%, more than 80%, or more than 90%, or more than 100% will be useful in the practice of the invention. In some implementations, such extracts will be derived from hydroponically grown *Tiliacora triandra* plant *(e.g.,* whole plant) or portion thereof (*e.g.*, vines, leaves, flowers, etc.). In some implementations, the HA stimulating extracts are obtained by extraction with an organic solvent, such as a polar (*e.g.*, ethanol, etc.) or non-polar solvent *(e.g.,* hexanes) and/or a protic (*e.g.,* alcohols, acids, etc.) or non-protic solvent (*e.g.*, dialkyl ethers, alkanes, esters, etc.). In some implementations, the extraction is carried out using a solvent system comprising from 5-50% (v/v) water and from 50-95% (v/v) ethanol, or comprising from 10-30% (v/v) water and from 70-90% (v/v) ethanol *(e.g.,* a 20/80 (v/v) water/ethanol solvent system).

In another aspect of the invention, a skincare composition is provided comprising any of the *Tiliacora triandra* extracts according to the invention. In one implementation, the extract of the *Tiliacora triandra* plant or portion thereof is derived from hydroponically grown *Tiliacora triandra* plant *(e.g.,* whole plant) or portion thereof *(e.g.,* vines, leaves, flowers, etc.), for example, using a solvent system comprising from 5-50% (v/v) water and from 50-95% (v/v) ethanol, or comprising from 10-30% (v/v) water and from 70-90% (v/v) ethanol *(e.g.,* a 20/80 (v/v) water/ethanol solvent system). In some implementations, the extract of a *Tiliacora triandra* plant or portion thereof is characterized by the ability of a 0.2% (w/w) solution of said extract to enhance levels of hyaluronic acid in fibroblast cells by more than 30% (or more than 40%, more than 50%, more than 60%, more than 70%, more than 80%, or more than 90%, or more than 100%). The extracts are typically dispersed in a physiologically compatible vehicle, such as a water-in-oil or oil-in-water emulsion. The vehicle will typically comprise from about 50-99% or 75-98% or about 80-95% by weight of the composition. The emulsions usually an emulsifier *(e.g.,* in an amount from about 0.01-10% by weight) and a preservative for microbial stability (*e.g.*, in an amount from about 0.0001-5% by weight). The extract is included in the formulation in an effective amount, by which is meant an amount adequate to impart a benefit to skin, but will typically be present in an amount from about 0.001-20% by weight or from about 0.01-10% by weight or from about 0.1-1% by weight of the composition. In some implementations the skincare composition will further comprise glycolic acid (or a salt thereof, such as sodium glycolate) in an amount from about 0.01-10% or from about 0.1-5% by weight. In some implementations, the weight ratio of the extract to glycolic acid ranges from about 10:1 to about 1:10 or from about 5:1 to about 1:5 or from about 3:1 to about 1:3 or from about 2:1 to about 1:2 or from about 3:2 to about 2:3 or about 1:1. In some implementations the skincare composition will further comprise niacinamide, for example, in an amount from about 0.01-10% or from about 0.1-5% by weight. In some implementations, the weight ratio of the extract to niacinamide ranges from about 50:1 to about 1:50 or from about 30:1 to about 1:30 or from about 25:1 to about 1:25 or from about 10:1 to about 1:10 or from about 5:1 to about 1:5 or from about 3:1 to about 1:3 or from about 2:1 to about 1:2 or from about 3:2 to about 2:3 or about 1:1. The skin care compositions of the invention may further comprise one or more cosmetic adjuvant or excipients, including without limitation, solvents, film formers, particulates, thickeners, rheology modifiers, surfactants and emulsifiers, stabilizers, pH modifiers, preservatives, chelators, colorants, and fragrances, each of which may be present individually or in the aggregate from about 0.0001-50% or 0.001-20% or 0.01-10% or 0.1-5% by weight of the composition. The skin care compositions of the invention may further comprise one or more skin active ingredients which impart a benefit to skin, including without limitation, minerals, vitamins (*e.g*., tocopherol acetate), retinoids (e.g., retinol, retinaldehyde, retinyl acetate, retinyl palmitate, etc.), antioxidants (*e.g.*, hexylresorcinol, phytol, thiodipropionic acid or di-lauryl esters thereof), alpha-hydroxy acids (*e.g.*, glycolic acid, lactic acid, citric acid, etc.), beta-hydroxy acids (*e.g.*, salicylic acid and esters thereof), peptides and palmitoyl derivatives thereof (*e.g.*, pentapeptide, hexapeptide, KTFK, K-ava-K, wheat protein hydrolysates, etc.), collagen boosters, collagenase inhibitors, elastin boosters, elastase inhibitors, hyaluronic acid boosters, skin plumpers (*e.g.*, hyaluronic acid), moisturizers, emollients *(e.g.,* dimethicone, vegetable oils, etc.), humectants *(e.g.,* glycerin), advanced glycation endproduct (AGE) inhibitors or cleavers, skin protectants, sunscreens and UV blockers, barrier repair and enhancing agents (*e.g.*, ceramides, glycerides, cholesterol and its esters, etc.), and botanicals, to name a few, each of which may be present individually or in the aggregate from about 0.0001-50% or 0.001-20% or 0.01-10% or 0.1-5% by weight of the composition.

In yet another aspect, a method is provided for improving the health of human skin or diminishing the appearance of dermatological signs of aging in human skin (*e.g.*, skin of the face, neck, chest, hands, etc.). The method typically comprises topically applying to an area of skin in need thereof (such as directly to a wrinkle or fine line) an effective amount of a skincare composition according to the invention. The skin care composition will include an effective amount of an extract of the *Tiliacora triandra* plant or portion thereof. For example, the extract may be derived from hydroponically grown *Tiliacora triandra* plant or portion thereof (*e.g.*, vines, leaves, flowers, etc.). The *Tiliacora triandra* plant extract may be characterized by the ability of a 0.2% (w/w) solution of the extract to enhance levels of hyaluronic acid in fibroblast cells by more than 30% (or more than 40%, more than 50%, more than 60%, more than 70%, more than 80%, or more than 90%, or more than 100%). In one implementation, the extract of the *Tiliacora triandra* plant or portion thereof is derived using a solvent system comprising from 5-50% (v/v) water and from 50-95% (v/v) ethanol, or comprising from 10-30% (v/v) water and from 70-90% (v/v) ethanol (*e.g*., a 20/80 (v/v) water/ethanol solvent system). The method is effective for diminishing the appearance of dermatological signs of aging, including:
(a) treatment, reduction, and/or prevention of fine lines or wrinkles;
(b) reduction of skin pore size;
(c) improvement in skin thickness, plumpness, and/or tautness;
(d) improvement in skin smoothness, suppleness and/or softness;
(e) improvement in skin tone, radiance, and/or clarity;
(f) improvement in procollagen, and/or collagen production;
(g) improvement in maintenance and remodeling of elastin;
(h) improvement in skin texture and/or promotion of retexturization;
(i) improvement in skin barrier repair and/or function;
(j) improvement in appearance of skin contours;
(k) restoration of skin luster and/or brightness;
(l) replenishment of essential nutrients and/or constituents in the skin;
(m) improvement of skin appearance decreased by aging and/or menopause;
(n) improvement in skin moisturization;
(o) increase in skin elasticity and/or resiliency;
(p) treatment, reduction, and/or prevention of skin sagging;
(q) improvement in skin firmness; and
(r) reduction of pigment spots and/or mottled skin; and
(s) improvement of optical properties of skin by light diffraction or reflection.

In one implementation, the method is for diminishing the appearance of wrinkles and/or fine lines, which may entail, for example, reducing the number of wrinkles and fine lines, reducing the depth of existing wrinkles and fine lines, forestalling the development or worsening of wrinkles and fine lines. In some implementations, the compositions are applied directly to a wrinkle and/or fine line, for example, at least once or twice daily for a period sufficient to note a visible improvement, such as at least one, two, four, eight, or twelve weeks or longer. In some embodiments, the compositions will be applied to the skin in an amount from about 0.001 to about 100 mg/cm², more typically from about 0.01 to about 20 mg/cm², or from about 0.1 to about 10 mg/cm² or from about 0.5 to about 5 mg/cm².

These and other aspects of the present invention will be better understood by reference to the following detailed description and appended claims.

### BRIEF DESCRIPTION OF THE DRAWINGS

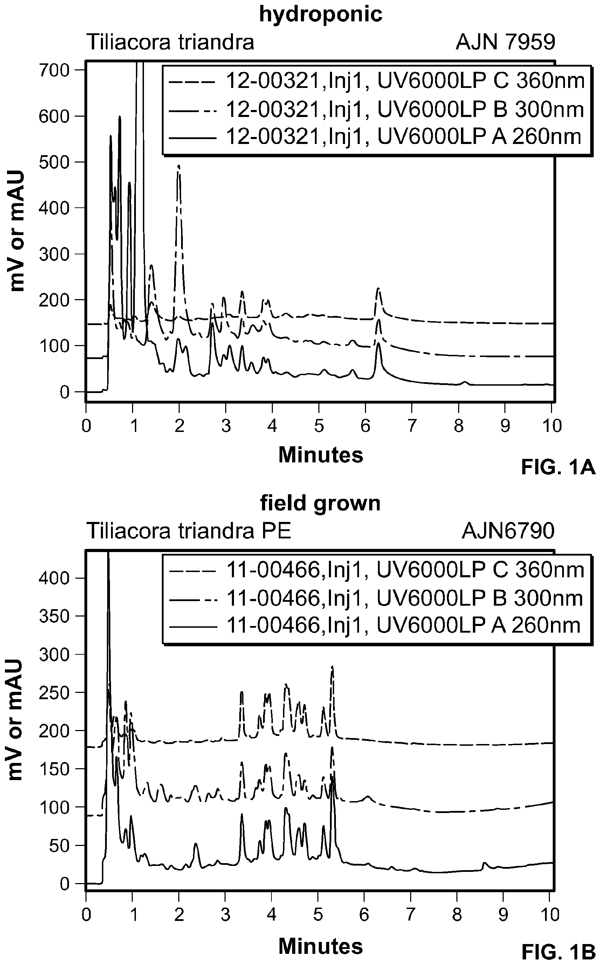

FIG. 1 is shows HPLC traces for extracts of *Tiliacora triandra.* FIG 1A shows a representative HPLC trace for a *Tiliacora triandra* extract prepared by extraction of hydroponically grown plant material using 20/80 (v/v) water/ethanol extraction solvent, and
FIG. 1B shows representative HPLC trace for a field grown *Tiliacora triandra* extract, prepared by extraction from field (*i.e.*, soil) grown plant using 20/80 (v/v) water/ethanol extraction solvent.

### DETAILED DESCRIPTION OF THE INVENTION

Detailed embodiments of the present invention are disclosed herein; however, it is to be understood that the disclosed embodiments are merely illustrative of the invention that may be embodied in various forms. In addition, each of the examples given in connection with the various embodiments of the invention is intended to be illustrative, and not restrictive. Therefore, specific structural and functional details disclosed herein are not to be interpreted as limiting, but merely as a representative basis for teaching one skilled in the art to variously employ the present invention.

All percentages given herein refer to the weight percentages of a particular component relative to the entire composition, including the vehicle, unless otherwise indicated. It will be understood that the sum of all weight % of individual components within a composition will not exceed 100%.

All terms used herein are intended to have their ordinary meaning unless otherwise provided. The phrases "cosmetically acceptable," "topically acceptable" and "dermatologically acceptable" are used interchangeably and are intended to mean that a particular component is generally regarding as safe and non-toxic for application to a human integument (*e.g.*, skin) at the levels employed. The term "prevent," as used herein, includes delaying or slowing the onset of or progression of a particular sign of skin aging. The phrase "individual in need thereof' refers to a human that could benefit from improved dermal appearance or health, including males or females. In some embodiments, the individual in need thereof is a female. The term "skin" includes, without limitation, the lips, skin of the face, hands, arms, neck, scalp, and chest. As used herein, the term "consisting essentially of' is intended to limit the invention to the specified materials or steps and those that do not materially affect the basic and novel characteristics of the claimed invention, as understood from a reading of this specification.

As used herein, the term "plant" included the whole plant or any portion therefore, uncles otherwise specified. Portions of the plant include, without limitation roots, vines, leaves, flowers, rhizomes, strobili, fruits, seeds, etc. The "aerial" portions or a plant include any portion that typically grows above ground in the corresponding field grown variety. The term "Tiliacora" refers to the genus that includes the species *Tiliacora dinklagei, Tiliacora funifera, Tiliacora gabonensis, Tiliacora kenyensis, Tiliacora racemosa,* and *Tiliacora triandra.* In some embodiments, any *Tilicaora* species in contemplated to be useful, provided the extract thereof is capable of up-regulating HA production in human fibroblasts (*e.g.*, by at least 30%). Whether, and to what extent, a particular extract up-regulates HA production is determined according to the protocol provided in Example 4. In some embodiments, any *Tilicaora* species in contemplated to be useful provided the extract thereof is obtained from a hydroponically grown plant or portion thereof, and demonstrates a skin benefit, for example, up-regulation of HA, pro-collagen, and/or collagen.

The present invention is founded on the discovery that extracts of hydroponically grown *Tiliacora triandra* plant are potent stimulators of HA and consequently are useful for skincare applications, such as methods for diminishing the appearance of wrinkles and fine lines in facial skin. By "hydroponic" is meant that the plant is grown substantially without contact between the roots and soil. Typically, hydroponic growth entails growth in a controlled environment, such as an indoor or greenhouse environment. The temperature, humidity, and pH are held substantially constant or maintained within narrow ranges as compared to field grown crops. Typically, the roots of the plant are continuously submerged in water of controlled temperature and pH. In some implementations, the roots may be embedded in a substrate, such as a clay aggregate, grow stone, coir peat, rice husks, perlite, vermiculite, pumice, sand, gravel, wood fiber, rock wool, sheep wool, brick shards, polystyrene, or other inert media, which is submerged in or contacted with water. The plants are continuously or periodically contacted with controlled amounts of nutrients, which, including without limitation, Ca²⁺ (calcium), Mg²⁺ (magnesium), K⁺ (potassium); NO₃⁻ (nitrate), SO₄⁻² (sulfate), and H₂PO₄⁻ (dihydrogen phosphate). The nutrients may be added to the water or may be delivered directly proximate the roots. The pH of the water is typically monitored and adjusted as necessary to between 4-8 or 5-7 or 5.5-6.5. Natural sunlight may be supplemented with light from metal-halide or high-pressure sodium lamps to lengthen the irradiation period or supplement natural light. Carbon dioxide may be injected into a sealed greenhouse environment to further promote growth. Unless otherwise indicated, the term "hydroponic" is not intended to be limited to a particular growth technique, other than substantially soilless conditions.

Any portion of the *Tiliacora* plant (*e.g., Tiliacora triandra*) may be used, including the whole plant, vines, leaves, flowers, etc. is provided. In some implementations, the extract is prepared from aerial portions of the plant, such as vines, leaves, and/or flowers. In one embodiment, the extract is prepared from vines. In one embodiment, the extract is prepared from leaves. Prior to extraction, the plant material is typically pulverized to facilitate extraction. The extract may be obtained using any known methodology. In one embodiment, the extract is obtained by steam distillation. In one embodiment, the extract is obtained by aqueous extraction, with an acidic, neutral, or basic pH. In one embodiment, the extract is obtained by extraction with an organic solvent. The organic solvent may include a polar (*e.g.*, ethanol, etc.) or non-polar solvent *(e.g.,* hexanes) and/or a protic (*e.g.*, alcohols, acids, etc.) or non-protic solvent (*e.g.*, dialkyl ethers, alkanes, esters, etc.), or mixtures thereof, or mixtures with water. In some implementations, the extraction is carried out using a polar protic solvent (*e.g.*, ethanol) alone or in combinations with water or other organic solvents. The plant material may be refluxed with the solvent or contacted with the solvent at ambient temperatures (*e.g.*, room temperature), above ambient temperatures, or below ambient temperatures. In one embodiment, the extract is prepared by extraction of the plant or a portion thereof with an ethanol, water, or water/ethanol extraction solvent. In one embodiment, the plant material is extracted with a solvent system comprising from 1-99% (v/v) water and from 1-99% (v/v) ethanol, or from 5-95% (v/v) water and from 5-95% (v/v) ethanol, or from 5-50% (v/v) water and from 50-95% (v/v) ethanol, or from 7.5-40% (v/v) water and from 60-92.5% (v/v) ethanol, or from 10-30% (v/v) water and from 70-90% (v/v) ethanol, or with an approximately 20/80 (v/v) water/ethanol solvent system.

The extract, including a hydroponic *Tiliacora triandra* extract *(e.g.,* extracted with water, ethanol, or water/ethanol, or ~ 20/80 (v/v) water/ethanol) may be applied directly to skin, but is typically included in a skin care formulation along with a vehicle or carrier.

The vehicle may comprise from about 20-99.99% by weight of the composition, but more typically will comprise from about 50-99.9% or 60-99% or from about 70-99% or from about 80-99% or from about 90-99% by weight of the composition. The vehicle may be, for example, in the form of a serum (*e.g.*, ethanolic or aqueous), gel, or emulsion.

Suitable emulsions include water-in-oil and oil-in-water. For purposes of this disclosure, silicone oil and waxes are regarded as "oil." Other emulsions, including without limitation, glycerin-in-oil or oil-in-glycerin emulsions, and multiple emulsions, are also contemplated to be useful. Emulsions will typically include an amount of an emulsifier suitable to stabilize the emulsion, for example, from about 0.001-20% by weight or 0.01-10% by weight or 0.1%-5% by weight. The emulsifier may be a nonionic, anionic or amphoteric surfactant.

The physiologically compatible vehicle may include water; vegetable oils; mineral oils; ester oils such as octal palmitate, isopropyl myristate and isopropyl palmitate; ethers such as dicapryl ether and dimethyl isosorbide; alcohols such as ethanol and isopropanol; fatty alcohols such as cetyl alcohol, cetearyl alcohol, stearyl alcohol and behenyl alcohol; isoparaffins such as isooctane, isododecane (IDD) and isohexadecane; silicone oils such as cyclomethicone, dimethicone, dimethicone cross-polymer, polysiloxanes and their derivatives, preferably organomodified derivatives including PDMS, dimethicone copolyol, dimethiconols, and amodimethiconols; hydrocarbon oils such as mineral oil, petrolatum, isoeicosane and polyolefins, e.g., (hydrogenated) polyisobutene; polyols such as propylene glycol, glycerin, butylene glycol, pentylene glycol, hexylene glycol, caprylyl glycol; waxes such as beeswax, carnauba, ozokerite, microcrystalline wax, polyethylene wax, and botanical waxes; or any combinations or mixtures of the foregoing. Aqueous vehicles, including serums, may include one or more solvents miscible with water, including lower alcohols, such as ethanol, isopropanol, and the like.

The skincare compositions may include one or more additional active agents, which may be present individually or in the aggregate, in an amount from about 0.001-50% by weight, more typically from about 0.01-20% by weight. As used herein, the term "active agent" included any agent that is intended to impart a benefit to skin. Additional active agents include, without limitation, minerals, vitamins, retinoids, antioxidants, alpha-hydroxy acids, beta-hydroxy acids, peptides, amino-acids, collagen boosters, collagenase inhibitors, elastin boosters, elastase inhibitors, hyaluronic acid boosters, skin plumpers, moisturizers, emollients, humectants, advanced glycation end-product (AGE) inhibitors or cleavers, keratolytic agents, desquamating agents, keratinocyte proliferation enhancers, 5-alpha reductase inhibitors, depigmenting agents, anti-inflammatory agents, steroids, anti-acne agents, skin protectants, sunscreens and UV blockers, botanicals, etc.

In one embodiment, the skincare composition will further comprise glycolic acid (or a salt thereof, such as sodium glycolate), for example, in an amount from about 0.01-10% or from about 0.1-5% by weight or from about 0.5-2.5% by weight, based on the weight of the entire composition. In some embodiments, the weight ratio of the *Tiliacora* extract (*e.g.*, hydroponic *Tiliacora triandra* extract) to glycolic acid ranges from about 10:1 to about 1:10 or from about 5:1 to about 1:5 or from about 3:1 to about 1:3 or from about 2:1 to about 1:2 or from about 3:2 to about 2:3 or about 1:1. In some embodiments, the combination of *Tiliacora triandra* extract and glycolic acid are capable of achieving a synergistic enhancement in HA production within fibroblasts, which is meant that the improvement is greater than additive.

In one embodiment, the skincare composition will further comprise niacinamide, for example, in an amount from about 0.01-10% or from about 0.1-5% by weight or from 0.1-1% by weight. In some embodiments, the weight ratio of the *Tiliacora* extract *(e.g.,* hydroponic *Tiliacora triandra* extract) to niacinamide ranges from about 50:1 to about 1:50 or from about 30:1 to about 1:30 or from about 25:1 to about 1:25 or from about 10:1 to about 1:10 or from about 5:1 to about 1:5 or from about 3:1 to about 1:3 or from about 2:1 to about 1:2 or from about 3:2 to about 2:3 or about 1:1. In some embodiments, the combination of *Tiliacora triandra* extract and niacinamide are capable of achieving a synergistic enhancement in HA production within fibroblasts, which is meant that the improvement is greater than additive.

In one embodiment, the skincare compositions will also include a retinoid. Exemplary retinoids include, without limitation, retinoic acid (*e.g.*, all-trans, or 9-cis, or 13-cis), and derivatives thereof, retinaldehyde, retinol (Vitamin A) and esters thereof, such as retinyl palmitate, retinyl acetate and retinyl propionate, and salts thereof. Particular mention may be made of retinol. When present, the retinoids will typically be included in amounts from about 0.0001% to about 5% by weight, more typically from about 0.01% to about 2.5% by weight, or from about 0.05% to about 1% by weight. Compositions according to this embodiment will typically include an antioxidant such as ascorbic acid and/or BHT and/or a chelating agent such as EDTA or a salt thereof (*e.g.*, disodium EDTA).

In some embodiments, a composition of the present invention will comprise phytol, for example in an amount about 0.001 percent by weight (wt %) to about 10 wt % based on the total weight of the composition. Typically, phytol may be present in an amount about 0.01 wt % to about 5 wt %, and most typically about 0.1 wt % to about 1 wt %, based on the total weight of the composition.

The composition may include any active for treating human skin, including for example, an active ingredient selected from glycolic acid, thiodipropionic acid (TDPA) or esters (*e.g.*, mono- and di-lauryl alcohol esters) thereof, hexylrecorcinol, niacinamide, or a botanical extract from the a plants of the genus *Eclipta* (e.g., *Eclipta* prostrata), *Portulaca* (*e.g.*, *Portulaca* grandiflora), or *Melicope* (*e.g., Melicope* hayesii and/or *Melicope* ellyarana), *Butea* (e.g., *Butea* frondosa); hydroxy acids (including alpha-hydroxy acids and beta-hydroxy acids), salicylic acid and alkyl salicylates; exfoliating agents (*e.g.*, glycolic acid, 3,6,9-trioxaundecanedioic acid, etc.), estrogen synthetase stimulating compounds (*e.g.*, caffeine and derivatives); compounds capable of inhibiting 5 alpha-reductase activity (*e.g.*, linolenic acid, linoleic acid, finasteride, and mixtures thereof); and barrier function enhancing agents alpha-hydroxy and omega-hydroxy fatty acids and esters thereof, etc.), to name a few. Additionally, an of the active ingredients, including botanicals, identified in U.S. Provisional patent applications 62/128,647, filed March 5, 2015, titled, "Methods For Treating Skin" are contemplated to be useful.

In another embodiment, the topical compositions of the present invention may also include one or more of the following: a skin penetration enhancer; an emollient, such as isopropyl myristate, petrolatum, volatile or non-volatile silicones oils (*e.g.*, methicone, dimethicone), ester oils, mineral oils, and fatty acid esters; a humectant, such as glycerin, hexylene glycol or caprylyl glycol; a skin plumper, such as palmitoyl oligopeptide, collagen, collagen and/or glycosaminoglycan (GAG) enhancing agents; a sunscreen, such as avobenzone or octyl methoxycinnamate; an exfoliating agent; and an antioxidant.

Suitable exfoliating agents include, for example, alpha-hydroxy acids, beta-hydroxy acids, oxa-acids, oxadiacids, and their derivatives such as esters, anhydrides and salts thereof. Suitable hydroxy acids include, for example, glycolic acid, lactic acid, malic acid, tartaric acid, citric acid, 2-hydroxyalkanoic acid, mandelic acid, salicylic acid and derivatives thereof. One exemplary exfoliating agent is glycolic acid. When present, the exfoliating agent may comprise from about 0.001% to about 20% by weight of the composition.

Examples of antioxidants that may be used in the present compositions include compounds having phenolic hydroxy functions, such as ascorbic acid and its derivatives/esters; beta-carotene; catechins; curcumin; ferulic acid derivatives (e.g., ethyl ferulate, sodium ferulate); gallic acid derivatives (e.g., propyl gallate); lycopene; reductic acid; rosmarinic acid; tannic acid; tetrahydrocurcumin; tocopherol and its derivatives, including tocopheryl acetate; uric acid; or any mixtures thereof. Other suitable antioxidants are those that have one or more thiol functions (-SH), in either reduced or non-reduced form, such as glutathione, lipoic acid, thioglycolic acid, and other sulfhydryl compounds. The antioxidant may be inorganic, such as bisulfites, metabisulfites, sulfites, or other inorganic salts and acids containing sulfur. Antioxidants may comprise, individually or collectively, from about 0.001% to about 10 % (w/w), or from about 0.01% to about 5% (w/w) of the total weight of the composition.

A sunscreen may be included to protect the skin from damaging ultraviolet rays. In an illustrative embodiment of the present disclosure, the sunscreen provides both UVA and UVB protection, by using either a single sunscreen or a combination of sunscreens. Among the sunscreens that can be employed in the present compositions are avobenzone, cinnamic acid derivatives (such as octylmethoxy cinnamate), octyl salicylate, oxybenzone, octocrylene, titanium dioxide, zinc oxide, or any mixtures thereof. The sunscreen may be present, individually or in the aggregate, from about 1 wt % to about 30 wt % of the total weight of the composition.

In one embodiment, the topical compositions will have a pH range from 1 to 13, with a pH in the range of from 2 to 12 being typical. In some embodiment, the composition will have a pH in the range of from 3.5 to 7 or from 7-10.5. In some embodiments, the pH will be in the range of 3-4, or 4-5, or 5-6, or 6-7, or 7-8, or 8-9, or 9-10, or 10-11, or 11-12. Suitable pH adjusters such as sodium hydroxide, citric acid and triethanolamine may be added to bring the pH within the desired range. Such pH adjusters will typically be present, individually or in the aggregate, from about 0.001 wt % to about 10 wt % of the total weight of the composition.

In addition, the compositions contemplated by this disclosure can include one or more compatible cosmetically acceptable adjuvants, such as colorants, pearls, pigments, optical diffusers, dyes, fragrances, preservatives, particulates, fillers, chelators, thickeners, antifungals, antimicrobials, antioxidants, film formers, insect repellents, photostabilizing agents, sunscreens, stabilizers, surfactants, thickeners, viscosity modifiers, gelling agents, and pH adjuster. Details with respect to these and other suitable cosmetic ingredients can be found in the "International Cosmetic Ingredient Dictionary and Handbook," 10th Edition (2004), published by the Cosmetic, Toiletry, and Fragrance Association (CTFA), at pp. 2177-2299. The amounts of these various substances are those that are conventionally used in the cosmetic or pharmaceutical fields, for example, they can constitute, individually or in the aggregate, from about 0.01% to about 20% of the total weight of the composition.

The compositions may be formulated in a variety of product forms, such as, for example, a lotion, cream, serum, spray, aerosol, cake, ointment, essence, gel, paste, patch, pencil, towelette, mask, stick, foam, elixir, concentrate, and the like, particularly for topical administration. The compositions are typically formulated as a lotion, cream, ointment, or gel.

The skincare compositions comprising extracts of *Tiliacora* (*e.g.*, hydroponically grown *Tiliacora triandra* extract, including such an extract obtained by extraction with a solvent system comprising from 5-50% water and 50-95% ethanol (v/v)) are intended for topical application to human integuments, including keratinous surfaces, such as skin, lips, nails, and hair. For purposes of this disclosure, the term "skin" includes lips. Typically, the compositions are topically applied to skin. The composition may be applied daily, every other day, weekly, etc., but maximum benefit is anticipated when they are applied at least once daily *(e.g.,* once or twice daily, including once daily in the morning or at night). The treatment is continued for a period of time sufficient to improve the health of skin and/or achieve a desired benefit of diminishing the signs of aging in the skin (*e.g.*, reduction in number or severity of wrinkles and/or fine lines, thickening thinning skin, or improving elasticity and/or diminishing sagging, etc.). This may entail topical application, at least once daily, for at least one week, or at least two weeks, or at least four weeks, or at least eight weeks or more. In some embodiments, the compositions are applied directly to a specific site of the skin (*i.e.*, directly onto a wrinkle and/or fine line, under the eyes, on a blemish, etc.). In some embodiments, the compositions will be applied to the skin in an amount from about 0.001 to about 100 mg/cm², more typically from about 0.01 to about 20 mg/cm², or from about 0.1 to about 10 mg/cm².

Numerous areas of the body can be treated, including, without limitation, the face, forehead, lips, scalp, neck, arms, hands, legs, knees, feet, chest, back, groin, buttocks, thighs, and the like. In some embodiments, the compositions are applied to the face, lips, chest, arms and/or hands, particularly, the face.

In certain embodiments of the invention, the compositions are applied topically to improve the appearance and/or health of human skin. The improvement in the appearance and/or health of human skin may be an improvement of any attribute or characteristic of skin, including without limitation:
(a) treatment, reduction, and/or prevention of fine lines or wrinkles;
(b) reduction of skin pore size;
(c) improvement in skin thickness, plumpness, and/or tautness;
(d) improvement in skin smoothness, suppleness and/or softness;
(e) improvement in skin tone, radiance, and/or clarity;
(f) improvement in procollagen, and/or collagen production;
(g) improvement in maintenance and remodeling of elastin;
(h) improvement in skin texture and/or promotion of retexturization;
(i) improvement in skin barrier repair and/or function;
(j) improvement in appearance of skin contours;
(k) restoration of skin luster and/or brightness;
(l) replenishment of essential nutrients and/or constituents in the skin;
(m) improvement of skin appearance decreased by aging and/or menopause;
(n) improvement in skin moisturization;
(o) increase in skin elasticity and/or resiliency;
(p) treatment, reduction, and/or prevention of skin sagging;
(q) improvement in skin firmness; and
(r) reduction of pigment spots and/or mottled skin; and
(s) improvement of optical properties of skin by light diffraction or reflection.

In some embodiments, the compositions are intended to treat winkles and/or fine lines in the skin, including forehead wrinkles, "crow's feet," and wrinkles at the edges of the eyes or mouth. In some embodiments, the compositions are applied directly to a wrinkle and/or fine line. The treatment may reduce the severity (*e.g*., depth) of the wrinkles and fine lines and/or may reduce the number of wrinkles and/or fine lines in a given area of skin. In some embodiments, the compositions are intended to treat sagging skin which may result from a loss of dermal elasticity. In this embodiment, the compositions may be applied to skin of the checks, jowls, etc.

It is also contemplated that the methods of the invention will be useful for treating thin skin by topically applying the composition to thin skin of an individual in need thereof. "Thin skin" is intended to include skin that is thinned due to chronological aging, menopause, or photo-damage and skin that is thinning prematurely. In some embodiments, the treatment is for thin skin in men, whereas other embodiments treat thin skin in women, pre-menopausal or post-menopausal, as it is believed that skin thins differently with age in men and women, and in particular in women at different stages of life.

The methods of the invention may be employed prophylactically to forestall aging including in individuals that have not manifested signs of skin aging, most commonly in individuals under 25 years of age. The methods may also reverse or treat signs of aging once manifested as is common in individuals over 25 years of age, or to slow the progression of dermatological aging in such individuals.

In certain embodiments, the cosmetic compositions described herein can be used to treat and/or prevent unwanted pigmentation or hyper-pigmentation of skin and/or of the hair, for example, to lighten skin or hair. In some embodiments, the compositions are topically applied to the skin or hair, for example to an area of hyper-pigmented skin or hair. Hyper-pigmentation includes any coloration of an individual's skin or hair that is darker than desired by the individual and that is caused by melanocytes. Such unwanted pigmentation may also be called discoloration. Hyper-pigmented areas of the skin include areas of discrete or mottled hyper-pigmentation. Areas of discrete hyper-pigmentation can be distinct, uniform areas of darker color and may appear as brown spots or freckles on the skin, including marks commonly called pigment spots or "age spots." Areas of mottled hyper-pigmentation of the skin can be dark blotches that are larger and more irregular in size and shape than areas of discrete pigmentation. Areas of hyper-pigmentation also include areas of tanned skin, for example, skin tanned due to UV exposure. Hyper-pigmented hair includes any shade of hair that is darker than desired.

Treating hyper-pigmentation or hyper-pigmented skin/hair refers to eradicating, reducing, ameliorating, or reversing one or more of the unwanted features associated with hyper-pigmentation, such as producing a perceptible lightening of the skin or hair in the affected area. Lightening hyper-pigmented areas of the skin may be desirable, in one embodiment, in diminishing age spots; lightening a suntan; evening or optimizing skin tones, e.g., in areas of mottled hyper-pigmentation; in treating melasmic and chloasmic patches, freckles, after-bum scars, and post-injury hyper-pigmentation. Preventing hyper-pigmentation or hyper-pigmented skin refers to affording skin, not yet affected by hyper-pigmentation, a benefit that serves to avoid, delay, forestall, or minimize one or more unwanted features associated with skin hyper-pigmentation, such as reducing the darkness or size of hyper-pigmented areas that eventually develop.

In one embodiment, the compositions of the invention are applied to human skin to reduce sebum production or improve the appearance of skin affected by cellulite, and/or reduce unwanted lipogenesis or increase lipolysis. In this embodiment, the compositions may include one or more additional agents such as anti-acne ingredients (*e.g.*, salicylic acid, benzoyl peroxide and other peroxides, sulfur, retinoids, etc.) in the case of a facial composition, or, in the case of a cellulite treatment, the formulation may comprise any ingredients suitable for treatment of cellulite, including without limitation, perilla oil and other unsaturated fatty oils and omega-3 fatty acids such as alpha-linolenic acid; caffeine; theophylline; xanthines; retinoids (*e.g.*, retinol); and the like. A cellulite treatment according to the invention will typically be applied topically to skin suffering from cellulite, including skin of the buttocks and thighs for a period of time sufficient to improve the appearance thereof, including for example, daily treatment for at least four weeks, at least eight weeks, at least twelve weeks, or longer. In one embodiment, the compositions are topically applied to treat acne.

In yet another aspect of the invention, a skincare composition comprising any of the *Tiliacora* extracts according to the invention (e.g., hydroponically grown *Tiliacora triandra* extract) are applied according to a treatment regimen comprising the steps of, in any order, (1) topically applying a skincare composition comprising *(e.g.,* hydroponic) *Tiliacora triandra* extract to the skin at least once daily for a first period of time (*e.g.*, from 1-31 day or from 2-15 days or from 5-10 days or 7 days), (2) topically applying a second skin care composition that does not comprise *Tiliacora triandra* extract for a second period of time *(e.g.,* from 1-31 day or from 2-15 days or from 5-10 days or 7 days), and (3) optionally repeating steps (1) and (2) for one or more (*e.g.*, at least four, five, six, seven, or eight times) or at least until a visible improvement in the health or appearance or dermatological signs or skin aging is noted or observed. In some implementations, the first and second periods of time are equal, for example, both may be one week. In some implementations, the second skincare composition will comprise effective amounts (*e.g.*, from about 0.001-10% or 0.01-5% or 0.1-5% by weight) of a retinoid (*e.g.*, retinol), phytol, thiodiproionic acid (or di-lauryl esters thereof), niacinamide, and/or glycolic acid. The first and second compositions may be packaged together or separately. When packaged together, they may be included in separate reservoirs within a single container, having two pumps for separately dispensing each composition. The packaged compositions may further include written instructions for, in any order, (1) topically applying a skincare composition comprising *(e.g.,* hydroponic) *Tiliacora triandra* extract to the skin at least once daily for a first period of time (*e.g.*, from 1-31 day or from 2-15 days or from 5-10 days or 7 days), (2) topically applying a second skin care composition that does not comprise *Tiliacora triandra* extract for a second period of time *(e.g.,* from 1-31 day or from 2-15 days or from 5-10 days or 7 days), and (3) optionally repeating steps (1) and (2) for one or more *(e.g.,* at least four, five, six, seven, or eight times) or at least until a visible improvement in the health or appearance or dermatological signs or skin aging is noted or observed.

In one embodiment, the compositions are intended for use as a non-therapeutic treatment. In another embodiment, the compositions are articles intended to be rubbed, poured, sprinkled, or sprayed on, introduced into, or otherwise applied to the human body for cleansing, beautifying, promoting attractiveness, or altering the appearance, in accordance with the US FD&C Act, §201(i).

### EXAMPLES

The following example illustrates a specific aspect of the instant description. The example should not be construed as limiting, as the example merely provides specific understanding and practice of the embodiments and its various aspects.

### EXAMPLE 1

### Hydroponic Tiliacora triandra plant growth.

A hydroponic *Tiliacora triandra* plant may be grown as follows. The stems of pest and disease free *Tiliacora triandra* plants are cut into pieces about 10 cm (4 inches) long. Each individual cutting comprises both a leaf and an eye. Two cuttings are placed in each cell of a 72 cell tray, where each cell contains a well-drained peat mix. Cuttings are held under mist in 70% shade. Some leaves are cut in half to reduce transpiration losses due to drying between mist cycles. Cuttings begin callusing in 7-10 days and are fully rooted in 40 days without the use of rooting hormone.

Two rooted cuttings are placed in a coir slab (available from Jiffy, Norway). Each coir slab should comprise two drainage slits in the bottom and two transplanting holes in the top. Coir slabs are expanded with a full strength nutrient solution (Arc Greenhouses, NJ) at a pH of 5.9.

Two drip irrigation emitters (Netafim, Israel) are placed at each plant location and used to deliver the nutrient solution directly to the roots in the coir slab. The delivery amount of nutrient solution is adjusted based on the amount of photosynthetically active radiation (PAR) received by the crops to maintain constant metabolic rate. During portions of the year in which sunlight is strongest, 30% ALUMINET shade cloth may be placed over crops. Daytime temperatures are maintained from about 23 °C (74°F) to about 29 °C (84°F) with good air movement over crops.

A trellis may be used to support growth. For example, two coir slabs may be placed end to end and a trellis built between the two rows of plants. Alternatively, the slabs may be suspended above ground with the plant foliage growing downward without the use of a trellis.

In all cases, if required, to control insect populations such as thrips, a mixture of 1.5 oz M-PEDE Insecticidal Soap (available from Gowan, AZ) per gallon water is sprayed on the plants every four days to reduce thrips infestation.

### EXAMPLE 2

### Extraction of hydroponic Tiliacora triandra.

An extract is obtained by extracting the vine of hydroponically grown *Tiliacora triandra* plant using an ethanol extraction scheme. Briefly, the vines of the dries plant are ground into small particles resulting in a powder. The ground powder is extracted with solvent comprising 80% ethanol/20% water (v/v). The solution is passed over charcoal to remove tannins and filtered. Solvent is removed by vacuum evaporation to yield a concentrated extract. The concentrated extract may be lyophilized. The extract is in the form of an oil having an "active" or solids content of approximately 50% (w/w).

### EXAMPLE 3

### HPLC of Tiliacora triandra extracts.

HPLC characterization of *Tiliacora triandra* extracts is carried out as follows. Samples were collected on a Thermo TSP HPLC system (P2000 Pump, AS 3000 Autosampler, SN 4000 controller, UV 6000 detector) with an Agilent Zorbax SB-C18 column (7.5 cm × 4.6 mm × 3.5 µm) at 40°C, and detection at 260, 300, and 360 nm. The injection volume was 20 µL with a flow rate of 1.5 mL/min with a gradient (v/v) as listed below in Table 1.

**Table 1.**

| Time (min) | Methanol | 1% Acetic Acid |
|---|---|---|
| 0 | 15% | 85% |
| 10 | 95% | 5% |
| 11 | 95% | 5% |
| 11.01 | 15% | 85% |
| 15 | 15% | 85% |

HPLC traces of (a) *Tiliacora triandra* extract, prepared by extraction of hydroponically grown plant using 20/80 (v/v) water/ethanol extraction solvent, and (b) field grown *Tiliacora triandra* extract, prepared by extraction from field (*i. e.*, soil) grown plant using 20/80 (v/v) water/ethanol extraction solvent, are shown in Figure 1(A) and (B), respectively.

### EXAMPLE 4

### Stimulation of HA production in human 3D skin tissues.

Method: Human 3D skin EFT400FT (MatTek, MA, USA) tissues were cultured following manufacture's instruction. *Tiliacora triandra* extract (extracted from hydroponically grown plant using 20/80 (v/v) water/ethanol) was added directly to the culture medium from a stock solution of 10% (w/w) extract in water in an amount adequate to achieve a final concentration of 0.2% (w/w) in the culture medium. The culture medium (EFT-400-MM) with and without *Tiliacora triandra* extract was collected 24 hours after dosing and fresh medium with and without *Tiliacora triandra* extract was added. Conditioned media from 3-day collection were pooled and assayed for the levels of HA.

HA production was measured using hyaluronic acid test kit (Corgenix, Inc. CO, USA). Briefly, 100 µl diluted conditioned medium was added to appropriate micro-wells and incubated for 60 minutes at room temperature. After the incubation is complete, the conditioned medium was removed and 100 µl HRP-conjugated HABP solution was added to each well and incubated for 30 minutes at room temperature. Wash each well 4 times with PBS after the incubation is complete. Add 100 µl One-component Substrate Solution to each well and incubate for 30 minutes at room temperature. Add 100 µl Stopping Solution to stop the enzyme reaction and read the O.D. of each well at 450 nm. The amount of HA in the conditioned medium was calculated from this standard curve. The stimulation of hyaluronic acid production is reported as percent change in hyaluronic acid level over the control, as shown below in Table 2.

**Table 2.**

| Treatment: | % change | *p* value |
|---|---|---|
| 0.2% (w/w) *Tiliacora triandra* (hydroponic) | 97.92 | 0.02 |
| 0.2% (w/w) *Tiliacora triandra* (field-grown) | 19.20 | 0.27 |

As shown above, the extract of hydroponically grown *Tiliacora triandra* at 0.2% (w/w) yielded a statistically significant (p < 0.05) increase in HA levels in fibroblast when topically applied to a 3D tissue model of human skin, whereas an otherwise identical extract from field grown *Tiliacora triandra* did not.

### EXAMPLE 5

### Synergistic stimulation of HA production in human 3D skin tissues.

The same procedure of Example 4 was repeated using the skincare active glycolic acid, alone or in combination with hydroponic *Tiliacora triandra* extract, to investigate possible synergies between hydroponic *Tiliacora triandra* extract and glycolic acid. *Tiliacora triandra* extract (extracted from hydroponically grown plant using 20/80 (v/v) water/ethanol) was applied to the culture medium in the same manner as in Example 4. Glycolic acid was formulated into the base formula provided below in Table 3 in an amount calculated to provide a 0.5% (w/w) concentration of glycolic acid in the emulsion. 30 µl of the 0.5% (w/w) glycolic acid emulsion was applied to the apical surface of the tissues in quadruple. The tissues were treated with the glycolic acid formulation for 8 hours per day for 3 consecutive days. At the end of treatment each day, test article was rinsed off with phosphate buffered saline.

**Table 3. Base formula**

| Ingredient: | Wt.% |
|---|---|
| Demineralized water | 57.8 |
| Disodium EDTA | 0.2 |
| Isopropyl palmitate | 35 |
| Cetearyl alcohol/Ceteareth-20 | 5 |
| Phenoxy ethanol | 0.5 |
| Imidazolidinyl urea | 0.5 |
| Hydroxyethyl acrylate/Sod. Acryloyldimethyl Copolymer Taurate/Isohexadecane/Polysorbate 60/AQ-BL | 1 |
| total | 100 |

The results are reported as percent change in HA level over the control, as shown below in Table 4.

**Table 4.**

| Treatment: | % change | *p* value |
|---|---|---|
| 0.5% (w/w) Glycolic acid | 25.21 | 0.06 |
| 0.2% (w/w) *Tiliacora triandra* extract (hydroponic) | 23.89 | 0.04 |
| 0.5% (w/w) Glycolic acid + 0.2% (w/w) *Tiliacora triandra* extract (hydroponic) | 72.86 | 0.03 |

As shown above, the combination of 0.5% (w/w) glycolic acid and 0.2% (w/w) extract of hydroponically grown *Tiliacora triandra* yielded a dramatic increase in HA levels in fibroblast when topically applied to a 3D tissue model of human skin, as compared to glycolic acid alone or the extract alone. It is believed that the combination of hydroponically grown *Tiliacora triandra* extract and glycolic acid produce a greater than additive, or synergistic, increase in HA levels in skin cells such as dermal fibroblasts. In some embodiments, hydroponically grown *Tiliacora triandra* extract and glycolic acid will be present in a skincare composition in a weight ratio of about 10:1 to about 1:10 or from about 5:1 to about 1:5 or from about 3:1 to about 1:3 or from about 5:2 to about 2:5 or from about 2:1 to about 1:2 or from about 3:2 to about 2:3 or about 1:1. In some embodiments, the aggregate amount of glycolic acid and extract of hydroponically grown *Tiliacora triandra* will range from about 0.1-10% or from about 0.1-7.5% or from about 0.2-5% or from about 0.5-2.5% by weight of the entire composition.

### EXAMPLE 6

### Synergistic stimulation of HA production in human 3D skin tissues.

The same procedure of Example 5 was repeated using the skincare active niacinamide, alone or in combination with hydroponic *Tiliacora triandra* extract, to investigate possible synergies between hydroponic *Tiliacora triandra* extract and niacinamide. *Tiliacora triandra* extract (extracted from hydroponically grown plant using 20/80 (v/v) water/ethanol) was applied to the culture medium in the same manner as in Example 4. Niacinamide was formulated into the base formula of Table 3 in an amount calculated to provide a 5% (w/w) concentration of niacinamide in the emulsion. 30 µl of the 5% (w/w) niacinamide emulsion was applied to the apical surface of the tissues in quadruple. The tissues were treated with the niacinamide formulation for 8 hours per day for 3 consecutive days. At the end of treatment each day, test article was rinsed off with phosphate buffered saline. The results are reported as percent change in HA level over the control, as shown below in Table 5.

**Table 5.**

| Treatment: | % change | *p* value |
|---|---|---|
| 5% (w/w) Niacinamide | 119.54 | 0.03 |
| 0.2% (w/w) *Tiliacora triandra* extract (hydroponic) | 98.51 | 0.04 |
| 5% (w/w) Niacinamide + 0.2% (w/w) *Tiliacora triandra* extract (hydroponic) | 306.03 | 0.01 |

As shown above, the combination of 5% (w/w) niacinamide and 0.2% (w/w) extract of hydroponically grown *Tiliacora triandra* plant yielded a dramatic increase in HA levels in fibroblast when topically applied to a 3D tissue model of human skin, as compared to niacinamide alone or the extract alone. It is believed that the combination of hydroponically grown *Tiliacora triandra* extract and glycolic acid produce a greater than additive, or synergistic, increase in HA levels in skin cells such as dermal fibroblasts. In some embodiments, hydroponically grown *Tiliacora triandra* extract and niacinamide will be present in a skincare composition in a weight ratio of about 100:1 to about 1:100 or from about 75:1 to about 1:75 or from about 50:1 to about 1:50 or from about 25:1 to about 1:25 or from about 10:1 to about 1:10 or from about 5:1 to about 1:5 or from about 2:1 to about 1:2 or from about 3:2 to about 2:3 or about 1:1. In some embodiments, the aggregate amount of niacinamide and extract of hydroponically grown *Tiliacora triandra* will range from about 0.1-10% or from about 0.1-7.5% or from about 0.2-5% or from about 0.5-2.5% by weight of the entire composition.

### EXAMPLE 7

### Stimulation of hyaluronic acid synthase (HAS) gene expression.

Total RNA was isolated from each treated 3D skin EFT400FT tissue using Trizol reagent (Thermo Fisher Scientific, MA). 1 µg of total RNA was used to generate cDNA using Supercript III first strand synthesis kit (Thermo Fisher Scientific, MA). Reverse transcriptase, Buffer, dNTP, Random primer, and RNase Inhibitor were diluted with the RNA according to the protocol from the manufacturer. 25ng cDNA was used in 20 µl q-PCR reactions. Briefly, 10 µl of TaqMan Advanced Master Mix (Thermo Fisher Scientific, MA), 4 µl of H₂O, 5 µl of cDNA, and 1 µl of either HAS primer (HAS2 Hs00193435_m1 and HAS3 Hs00193436_ml, Applied Biosystems) or 18S (Hs99999901_s1, Applied Biosystems) as a house keeping gene were mixed in a 96 well plate. The condition of qPCR was an incubation step of 95°C for 20 seconds followed by 40 cycles of 95°C for 3 seconds and 60°C for 30 seconds. CT and RQ values were obtained from the software of applied Biosystems 7500 Fast Real Time PCR system. The results are shown below in Table 6, as percentage change of expression of hyaluronan synthase genes over control.

**Table 6.**

| | *HAS2* gene | *HAS3* gene |
|---|---|---|
| Treatment: | % change | % change |
| Glycolic acid | No effect | 101 |
| Niacinamide | No effect | 106 |
| *Tiliacora triandra* extract (hydroponic) | 496 | 112 |

As shown in Table 6, hydroponic *Tiliacora triandra* extract, like glycolic acid and niacinamide, upregulates expression of the *HAS3* gene for hyaluronan synthase. However, unlike glycolic acid and niacinamide, the hydroponic *Tiliacora triandra* extract also upregulates the *HAS2* gene for hyaluronan synthase. In fact, hydroponic *Tiliacora triandra* extract is such a potent upregulator of *HAS2* gene expression that nearly a 5-fold increase was observed. Based on this data, it is believed that the mode of action of hydroponic *Tiliacora triandra* extract is complementary to and distinct from that of glycolic acid, niacinamide, or other upregulators of the *HAS2* and/or *HAS3* gene.

As various changes can be made in the above-described subject matter without departing from the scope of the present invention, it is intended that all subject matter contained in the above description be interpreted as descriptive and illustrative of the present invention. Many modifications and variations of the present invention are possible in light of the above teachings. Accordingly, the present description is intended to embrace all such alternatives, modifications, and variances which fall within the scope of the appended claims.

## Claims

1. An extract of a hydroponically grown *Tiliacora triandra* plant or portion thereof.

2. The extract according to claim 1, wherein said extract is prepared from aerial portions of the plant, and wherein said extract is optionally prepared from:
(i) leaves of the plant; or
(ii) vines of the plant.

3. The extract according to claim 1, prepared by extraction of said plant or a portion thereof with a polar protic solvent.

4. The extract according to claim 3, prepared by extraction of said plant or a portion thereof with a water/ethanol extraction solvent, wherein optionally:
(i) the solvent system comprises from 5-50% (v/v) water and from 50-95% (v/v) ethanol; or
(ii) the solvent system comprises from 10-30% (v/v) water and from 70-90% (v/v) ethanol.

5. A skincare composition comprising from 0.001- 10% by weight of the *Tiliacora triandra* plant extract according to claims 1-4, dispersed in a physiologically compatible vehicle, wherein optionally said vehicle is in the form of a water-in-oil or oil-in-water emulsion comprising from 0.01-10% by weight of an emulsifier and a preservative.

6. The skincare composition according to claim 5, wherein further comprising glycolic acid in an amount from 0.01-10% by weight and where the weight ratio of said extract to said glycolic acid ranges from 5:1 to 1:5.

7. The skincare composition according to claim 5, wherein further comprising niacinamide in an amount from 0.01-10% by weight and where the weight ratio of said extract to said niacinamide ranges from 50:1 to 1:5.

8. A non-therapeutic method for improving the health of human skin or diminishing the appearance of dermatological signs of aging in human skin comprising topically applying to an area of skin in need thereof an effective amount of a skincare composition comprising an extract of a hydroponically grown *Tiliacora triandra* plant or portion thereof in a physiologically compatible vehicle.

9. The non-therapeutic method according to claim 8, wherein said extract of a hydroponically grown *Tiliacora triandra* plant or portion thereof **characterized by** the ability of a 0.2% (w/w) solution of said extract to enhance levels of hyaluronic acid in fibroblast cells by more than 30%.

10. The non-therapeutic method according to claim 8, wherein said extract is prepared by extraction of said plant or a portion thereof with a solvent system comprising from 10-30% (v/v) water and from 70-90% (v/v) ethanol.

11. The non-therapeutic method according to claim 8, wherein the diminishing the appearance of dermatological signs of aging is selected from a group consisting of:
(a) treatment, reduction, and/or prevention of fine lines or wrinkles;
(b) reduction of skin pore size;
(c) improvement in skin thickness, plumpness, and/or tautness;
(d) improvement in skin smoothness, suppleness and/or softness;
(e) improvement in skin tone, radiance, and/or clarity;
(f) improvement in procollagen, and/or collagen production;
(g) improvement in maintenance and remodeling of elastin;
(h) improvement in skin texture and/or promotion of retexturization;
(i) improvement in skin barrier repair and/or function;
(j) improvement in appearance of skin contours;
(k) restoration of skin luster and/or brightness;
(l) replenishment of essential nutrients and/or constituents in the skin;
(m) improvement of skin appearance decreased by aging and/or menopause;
(n) improvement in skin moisturization;
(o) increase in skin elasticity and/or resiliency;
(p) treatment, reduction, and/or prevention of skin sagging;
(q) improvement in skin firmness; and
(r) reduction of pigment spots and/or mottled skin; and
(s) improvement of optical properties of skin by light diffraction or reflection.

12. The non-therapeutic method according to claim 8, wherein the diminishing the appearance of dermatological signs of aging comprises reducing the appearance of wrinkles and/or fine lines.

13. The non-therapeutic method according to claim 8, wherein said skin is skin of the face.

## Patentansprüche

1. Extrakt einer hydroponisch angebauten *Tiliacora triandra-*Pflanze oder eines Teils davon.

2. Extrakt nach Anspruch 1, wobei der Extrakt aus den Luftteilen der Pflanze hergestellt wird, und wobei der Extrakt optional aus
(i) den Blättern der Pflanze; oder
(ii) den Reben der Pflanze
hergestellt wird.

3. Extrakt nach Anspruch 1, der durch Extraktion der Pflanze oder eines Teils der Pflanze mit einem polaren protischen Lösemittel hergestellt wird.

4. Extrakt nach Anspruch 3, der durch Extraktion der Pflanze oder eines Teils der Pflanze mit einem Wasser/Ethanol-Extraktionslösemittel hergestellt wird, wobei optional
(i) das Lösemittelsystem von 5% bis 50% (v/v) Wasser und von 50% bis 95% (v/v) Ethanol enthält; oder
(ii) das Lösemittelsystem von 10% bis 30% (v/v) Wasser und von 70% bis 90% (v/v) Ethanol enthält.

5. Hautpflegeverbindung, die von 0,001 Gewichtsprozent bis 10 Gewichtsprozent des *Tiliacora triandra*-Pflanzenextrakts nach Ansprüchen 1 bis 4 enthält, das in einem physiologisch kompatiblen Vehikel dispergiert ist, wobei optional das Vehikel in der Form einer Wasser-in-Öl- oder Öl-in-Wasser-Emulsion, die von 0,01 Gewichtsprozent bis 10 Gewichtsprozent eines Emulgators und eines Konservierungsmittels enthält, ist.

6. Hautpflegeverbindung nach Anspruch 5, wobei die Hautpflegeverbindung weiter Glycolsäure in einer Menge von 0,01 Gewichtsprozent bis 10 Gewichtsprozent enthält und wobei der Gewichtsanteil des Extrakts zur Glycolsäure zwischen 5:1 bis 1:5 ist.

7. Hautpflegeverbindung nach Anspruch 5, wobei die Hautpflegeverbindung weiter Nikotinamid in einer Menge von 0,01 Gewichtsprozent bis 10 Gewichtsprozent enthält und wobei der Gewichtsanteil des Extrakts zum Nikotinamid zwischen 50:1 und 1:5 ist.

8. Nicht-therapeutisches Verfahren zur Verbesserung der Gesundheit der menschlichen Haut oder zur Verminderung von dermatologischen Anzeichen des Alterns der menschlichen Haut, das das topische Anwenden einer effektiven Menge einer Hautpflegeverbindung, die ein Extrakt einer hydroponisch angebauten *Tiliacora triandra-*Pflanze oder eines Teils davon in einem physiologisch kompatiblen Vehikel enthält, auf eine Fläche der Haut, die es nötig hat.

9. Nicht-therapeutisches Verfahren nach Anspruch 8, wobei der Extrakt einer hydroponisch angebauten *Tiliacora triandra-*Pflanze oder eines Teils davon dadurch charakterisiert ist, dass eine 0,2% (w/w) Lösung des Extrakts das Niveau von Hyaluronsäure in Fibroblasten um mehr als 30% erhöht.

10. Nicht-therapeutisches Verfahren nach Anspruch 8, wobei der Extrakt durch Extraktion der Pflanze oder eines Teils der Pflanze mit einem Lösemittelsystem, das von 10% (v/v) bis 30% (v/v) Wasser und von 70% (v/v) bis 90% (v/v) Ethanol enthält, hergestellt wird.

11. Nicht-therapeutisches Verfahren nach Anspruch 8, wobei die Verringerung der dermatologischen Anzeichen des Alterns ausgewählt ist aus der Gruppe bestehend aus:
(a) Behandlung, Verringerung, und/oder Vorbeuge feiner Linien oder Falten;
(b) Verringerung der Hautporengröße;
(c) Verbesserung der Hautdicke, -plumpheit, und/oder -straffheit;
(d) Verbesserung der Hautglätte, -geschmeidigkeit, und/oder -weichheit;
(e) Verbesserung des Hauttons, der Hautstrahlung und/oder der Hautklarheit;
(f) Verbesserung der Procollagen- und/oder Collagenproduktion;
(g) Verbesserung des Erhalts und Umbaus von Elastin;
(h) Verbesserung der Hauttextur und/oder Förderung der Retexturierung;
(i) Verbesserung der Reparatur der Hautbarriere und/oder deren Funktion;
(j) Verbesserung des Erscheinungsbilds von Hautkonturen;
(k) Wiederherstellung der Hautglanzes und der Hauthelligkeit;
(l) Wiederauffüllen essentieller Nährstoffe und/oder Bestandteile der Haut;
(m) Verbesserung des Erscheinungsbildes der Haut, das durch Altern und/oder die Menopause verschlechtert wurde;
(n) Verbesserung der Hautfeuchtigkeit;
(o) Verbesserung der Hautelastizität und/oder -widerstandsfähigkeit;
(p) Behandlung, Verringerung und/oder Vorbeuge von Hautschlaffheit;
(q) Verbesserung der Hautfestigkeit; und
(r) Verringerung von Pigmentflecken und/oder gefleckter Haut; und
(s) Verbesserung der optischen Eigenschaften der Haut unter Lichtbrechung oder -reflexion.

12. Nicht-therapeutisches Verfahren nach Anspruch 8, wobei die Verringerung der dermatologischen Anzeichen des Alterns die Reduktion des Erscheinens von Falten und/oder feinen Linien enthält.

13. Nicht-therapeutisches Verfahren nach Anspruch 8, wobei die Haut Gesichtshaut ist.

## Revendications

1. Extrait d'une plante de *Tiliacora triandra* cultivée de façon hydroponique ou d'une partie de celle-ci.

2. Extrait selon la revendication 1, dans lequel ledit extrait est préparé à partir de parties aériennes de la plante, et dans lequel ledit extrait est facultativement préparé à partir de :
(i) feuilles de la plante ; ou
(ii) lianes de la plante.

3. Extrait selon la revendication 1, préparé par extraction de ladite plante ou d'une partie de celle-ci avec un solvant protique polaire.

4. Extrait selon la revendication 3, préparé par extraction de ladite plante ou d'une partie de celle-ci avec un solvant d'extraction eau/éthanol, dans lequel facultativement :
(i) le système de solvant comprend de 5 à 50 % (v/v) d'eau et de 50 à 95 % (v/v) d'éthanol ; ou
(ii) le système de solvant comprend de 10 à 30 % (v/v) d'eau et de 70 à 90 % (v/v) d'éthanol.

5. Composition de soins cutanés comprenant de 0,001 à 10 % en poids de l'extrait de plante *Tiliacora triandra* selon les revendications 1 à 4, dispersée dans un support physiologiquement compatible, dans laquelle facultativement ledit support se présente sous la forme d'une émulsion d'eau dans l'huile ou d'huile dans l'eau comprenant de 0,01 à 10 % en poids d'un émulsifiant et d'un conservateur.

6. Composition de soins cutanés selon la revendication 5, comprenant en outre de l'acide glycolique dans une quantité allant de 0,01 à 10 % en poids et dans laquelle le rapport en poids dudit extrait audit acide glycolique s'étend de 5:1 à 1:5.

7. Composition de soins cutanés selon la revendication 5, comprenant en outre du niacinamide dans une quantité allant de 0,01 à 10 % en poids et dans laquelle le rapport en poids dudit extrait audit niacinamide s'étend de 50:1 à 1:5.

8. Procédé non-thérapeutique d'amélioration de la santé de la peau humaine ou de diminution de l'apparition des signes dermatologiques du vieillissement de la peau humaine comprenant l'application locale à une zone de la peau en ayant besoin d'une quantité efficace d'une composition de soins cutanés comprenant un extrait d'une plante de *Tiliacora triandra* cultivée de façon hydroponique ou d'une partie de celle-ci dans un véhicule physiologiquement compatible.

9. Procédé non-thérapeutique selon la revendication 8, dans lequel ledit extrait d'une plante de *Tiliacora triandra* cultivée de façon hydroponique ou d'une partie de celle-ci se **caractérise par** la capacité d'une solution à 0,2 % (m/m) dudit extrait à améliorer les niveaux d'acide hyaluronique dans les cellules de fibroblastes de plus de 30 %.

10. Procédé non-thérapeutique selon la revendication 8, dans lequel ledit extrait est préparé par extraction de ladite plante ou d'une partie de celle-ci avec un système de solvant comprenant de 10 à 30 % (v/v) d'eau et de 70 à 90 % (v/v) d'éthanol.

11. Procédé non-thérapeutique selon la revendication 8, dans lequel la diminution de l'apparition des signes dermatologiques du vieillissement est sélectionnée dans un groupe constitué par :
(a) le traitement, la réduction, et/ou la prévention des rides ou ridules ;
(b) la réduction de la taille des pores de la peau ;
(c) l'amélioration de l'épaisseur, du volume, et/ou de la tonicité de la peau ;
(d) amélioration de la douceur, de la souplesse et/ou du velouté de la peau ;
(e) amélioration du teint, de l'éclat et/ou de la clarté de la peau ;
(f) amélioration de la production de procollagène, et/ou de collagène ;
(g) amélioration du maintien et du remodelage de l'élastine ;
(h) amélioration de la texture de la peau et/ou de la promotion de la restructuration ;
(i) amélioration de la réparation et/ou de la fonction de barrière de la peau ;
(j) amélioration de l'aspect des contours de la peau ;
(k) restauration de la brillance et/ou de la luminosité de la peau ;
(l) régénération des nutriments et/ou constituants essentiels de la peau ;
(m) amélioration de l'aspect de la peau dégradé par le vieillissement et/ou la ménopause ;
(n) amélioration de l'hydratation de la peau ;
(o) augmentation de l'élasticité et/ou de la résilience de la peau ;
(p) traitement, réduction, et/ou prévention du relâchement cutané ;
(q) amélioration de la fermeté de la peau ; et
(r) réduction des taches pigmentaires et/ou de la peau tachetée ; et
(s) amélioration des propriétés optiques de la peau par diffraction ou réflexion de la lumière.

12. Procédé non-thérapeutique selon la revendication 8, dans lequel la diminution de l'apparition des signes dermatologiques du vieillissement comprend la réduction de l'apparition de rides et/ou de ridules.

13. Procédé non-thérapeutique selon la revendication 8, dans lequel ladite peau est la peau du visage.
